# EUROPEAN PATENT APPLICATION

(11) **EP 4 480 488 A1**
(43) Date of publication of application: **25.12.2024**
(21) Application number: 23774055.0
(22) Date of filing: 14.03.2023
(51) Int. Cl.: A61K 36/81, A61P 1/02, A61K 45/00

(54) **PHARMACOLOGICAL, HEALTH OR DIETARY PREPARATION FOR TREATING HYPOSALIVATION**

(30) Priority: 22.03.2022 ES 202230252
(71) Applicant: De Miguel Lara, Antonio, 41013 Sevilla (ES)
(72) Inventor: De Miguel Lara, Antonio, 41013 Sevilla (ES)
(74) Representative: Urizar Anasagasti, Jesus Maria
(86) International application number: PCT/ES2023/070146
(87) International publication number: WO 2023/180597

(57) **Abstract**

Pharmacological, sanitary, or dietary preparation for use in the treatment of low salivary secretion, which includes any *Withania somnifera* (ashwagandha) plant extract, obtained by means of any method and at any concentration. The use of the *Withania somnifera* (ashwagandha) plant is intended for the treatment of low salivary secretion by the pharmacological action of the extract from any part of the plant.

## Description

### Technical sector

The sector in which the present invention falls is the medical sciences and hygiene sector, more specifically in the sector of preparations for medical, dental, or toiletry purposes, for the care of teeth, oral cavity, or dentures; mouthwashes and medicinal preparations based on medicinal plants or herbal medicines.

### State of the art

To try to alleviate the problem of dry mouth or xerostomia, it can be done by alleviating the symptoms of dry mouth or xerostomia or by alleviating the problem of the lack of or decreased secretion of saliva that gives rise to this dry mouth, and the patent literature includes several remedies based on the use of plants, namely:
US2022062157 describes products that include one or more plant based oils, in addition to a mucoadhesive polymer. Among the articles used to prevent formation or maintenance of a biofilm, loss of hydration or lubricity in oral tissue, or to treat xerostomia or dry mouth, the following are included: pullulan, xanthan gum, guar gum, ethyl cellulose, and carrageenan.

CN111956741 discloses a plant composition for promoting saliva secretion and relieving xerostomia, comprising dendrobium nobile, Chinese prickly ash, schisandra chinensis, and liquorice.

EP3656370 relates to a pharmaceutical composition comprising lysozyme, dextranase, and an essential oil of a plant belonging to the family Myrtaceae for the prevention and treatment, among other oral conditions, of xerostomia.

US2017151303 provides various compositions and methods to alleviate one or more symptoms associated with hyposalivation and/or xerostomia, which employ one or more plant pulp products and/or a proanthocyanidin at a concentration effective to reduce or eliminate the symptoms.

CN105709116 provides an herbaceous plant composition that is prepared from the seeds of Job's tears, radix polygonati officinalis, radix ophiopogonis, chrysanthemum, honeysuckle, bamboo leaves, radix astragali and Goji berry fruits. This composition is used to alleviate the symptoms of xerostomia, inter alia.

Patent document US2004191388 is the closest prior art document, and it relates to a composition useful as a hydrating beverage comprising at least one complex carbohydrate, at least one chelated electrolyte, betaine, and piperine. This document refers to the fact that it can be used for the treatment of xerostomia along with other disorders derived from dehydration, precisely because it is a useful product as a hydrating beverage. It also refers to the fact that the product has essential active ingredients detailed in claim 1 thereof, which are at least one complex carbohydrate, at least one chelated electrolyte, betaine, and piperine. Additionally, it is mentioned that this hydrating beverage may include in its composition plant extracts such as ashwagandha, which is the common name for *Withania somnifera.* At no point is there any reference to the product being used to treat xerostomia due to the use of ashwagandha, and reference is made to the use of this plant or other plant extracts as if they were additives. This is logical since the product of this patent is a useful product as a hydrating beverage and as such, with dry mouth, a hydrating effect is obviously helpful. Unlike this patent, the invention described in this specification is used in specific cases of xerostomia, but not for its hydrating effect, but because the extract of this plant has a direct effect in cases of low salivary secretion, promoting the secretion of saliva and, consequently, eliminating dry mouth. The mechanism of both is completely different since in one case, dry mouth is controlled by means of hydration, and in the case of the present invention, it is controlled by means of increasing salivation and, consequently, it has a longer lasting effect.

*Withania Somnifera* (ashwagandha) is considered an adaptogen by the European Medicines Agency, but its use to alleviate the symptoms of xerostomia or low salivary secretion has not been described in the medical literature.

The concept of adaptogens is already more than 60 years old, and they have been defined as substances that increase resistance to a broad spectrum of harmful factors (stressors) of a different physical, chemical, and biological nature. Adaptogens are considered metabolic regulators that increase the body's ability to adapt to environmental factors and avoid damage caused by these factors. *Withania somnifera* has been used for thousands of years to promote physical and mental health, improve the body's defense mechanisms, and increase longevity.

The pharmacology of adaptogens is a typical example of network pharmacology. They do not act on a single receptor but exhibit multi-target action and the shared use of a number of different receptors. This type of pharmacology has the potential to provide treatment for complex diseases, chronic conditions, and syndromes, including its pathophysiological evolution where conventional approaches have often been disappointing.

Clinical trials and animal research support the use of *Withania somnifera* for anxiety, cognitive and neurological disorders, inflammation, and Parkinson's disease. *Withania somnifera* is also used therapeutically as an adaptogen for patients with nervous exhaustion, insomnia, and weakness due to stress, and as an immune stimulant in patients with low white blood cell counts.

According to clinical research, *Withania somnifera* has anti-stress, antioxidant, antiinflammatory, and antitumor activity. It also has immunomodulatory actions, inhibition of cognitive deficits in animal models of Alzheimer's disease, and anxiolytic and antidepressant action demonstrated in tests on rats. It was also reported that *Withania somnifera* has significant effects on brain neurotransmitter functions in rats: https://www.researchgate.net/figure/Neuroprotective-mechanisms-of-Withania-somnifera fig5 273003949.

The roots are the main part used therapeutically. The pharmacological activity thereof is attributed to alkaloids and steroidal lactones. Twelve alkaloids, 35 withanolides, and several sitoindosides have been isolated. The pharmacological activity of *Withania somnifera* has been attributed primarily to withaferin A and withanolide D.

### Description of the invention

In the development of the invention, it has been proven that one of the most important mechanisms of action of *Withania somnifera* consists of an increase in the concentration of the neurotransmitter acetylcholine in the synaptic gap.

Acetylcholine is released into the synapse in response to a specific stimulus. The secreted neurotransmitter acts on highly selective receptors located in postsynaptic cells; this causes changes in their metabolism and modifies their cellular activity.

Acetylcholine is widely distributed in the central nervous system and is involved in memory circuits, reward circuits, extrapyramidal circuits, in the peripheral nervous system, and in the autonomic nervous system.

When acetylcholine binds to many receptors on the motor end plate of muscle fibers, it produces excitatory postsynaptic potentials, which result in the generation of an action potential in the muscle fiber with its corresponding contraction. Glands that receive impulses from the parasympathetic part of the autonomic nervous system are stimulated in the same way. For this reason, it has been observed that an increase in acetylcholine causes a reduction in heart rate and an increase in saliva production.

With increasing age, there is a decrease in acetylcholine in the synaptic gap. As a result of this, heart rate may increase, there is memory loss, increased anxiety, etc.

*Withania somnifera* acts by activating the enzyme choline acetyltransferase, which is responsible for its synthesis, or by inhibiting acetylcholinesterase, which is responsible for its destruction; as a consequence, the concentration of acetylcholine in the synaptic gap increases. About half of the acetylcholine is broken down before reaching the receptors on the postsynaptic membrane, which is its target, and molecules that reach the receptors are generally broken down within 20 milliseconds of their arrival. It has been observed that a decrease in the action of acetylcholine is related to a decrease in the secretion of saliva flow.

Dry mouth or xerostomia generally occurs with age and the consumption of many drugs (80% of the most prescribed drugs). Most of the mechanisms by which drugs promote decreased salivation are related to an anticholinergic action. It is important to note that the salivary gland can atrophy over time if the nerve stimuli to activate saliva flow are not produced.

The action of *Withania somnifera* (ashwagandha) on saliva production consists of increasing the activity of acetylcholine on the salivary glands. As a result, saliva flow increases.

Based on the prior art, the objective of the present invention is to provide a new composition that, when used in the treatment of xerostomia, allows the activity of acetylcholine on the salivary glands to increase, in order to increase saliva flow and thus reduce the effects of xerostomia, produced by any cause.

This preparation includes any *Withania somnifera* (ashwagandha) plant extract, obtained by means of different methods (aqueous extract, ethanol extract, etc.) and at any concentration which is valid for the intended purpose.

There is also claimed a new indication for *Withania somnifera* (ashwagandha) for use in the production of preparations, which include mouthwashes, sprays, toothpaste, etc., which are pharmacological or sanitary products, as well as tablets or other Galenic forms intended for the treatment of dry mouth due to the lack of or decrease in saliva secretion, which is alleviated by the pharmacological action of the extract from any part of the plant and at any concentration.

## Claims

1. A pharmacological, sanitary, or dietary preparation for the treatment of low salivary secretion, **characterized in that** it includes any *Withania somnifera* (ashwagandha) plant extract, obtained by means of any method and at any concentration.

2. Use of the *Withania somnifera* (ashwagandha) plant for the production of pharmacological preparations, medical devices, or dietary supplements for the treatment of low salivary secretion.
